# EUROPEAN PATENT APPLICATION

(11) **EP 1 849 490 A1**
(43) Date of publication of application: **31.10.2007**
(21) Application number: 05719121.5
(22) Date of filing: 15.02.2005
(51) Int. Cl.: A61M 5/315

(54) **SYRINGE**

(71) Applicant: KABUSHIKI KAISHA TOP, Tokyo 120-0035 (JP)
(72) Inventor: Chiba, Atsushi, Adachi-ku, Tokyo 1200035 (JP); Manaka, Toshiyuki, Adachi-ku, Tokyo 1200035 (JP)
(74) Representative: Henkel, Feiler & Hänzel
(86) International application number: PCT/JP2005/002219
(87) International publication number: WO 2006/087762

(57) **Abstract**

A syringe in which liquid leakage can be prevented even if a gasket is subjected to high pressure is provided. The present invention includes a gasket 3 that freely slides inside a barrel 2, in which a lure tapered section 5 is formed, to seal it and has a cavity section 10 in an inner periphery side; and a plunger rod 4. The plunger rod 4 is coupled with the gasket 3 through an anchor section 11. The anchor section 11 is projected from a pedestal section 18 having a tapered surface inclined at 5 degrees to 45 degrees relative to a plane orthogonal to the axial direction of the plunger rod 4, has a head contact section 22 coming into contact with the backside of the head of the gasket 3, and has a contact member 12 that is loosely fitted on the outer periphery side and is in contact with the inner peripheral wall of the gasket 3. The pedestal section 18 has a curved recess surface 20 between the head contact section 22 and the tapered surface, and the contact member 12 has a curved projection section 28a at a position facing the curved recess surface 20. The barrel 2 has a lock tube 6 having a screw thread 7 formed on its inner periphery side. The syringe 1 is used for injection of a contrast agent.

## Description

### Technical Field

The present invention relates to a syringe used in medical practice such as injection of a contrast medium or the like.

### Background Art

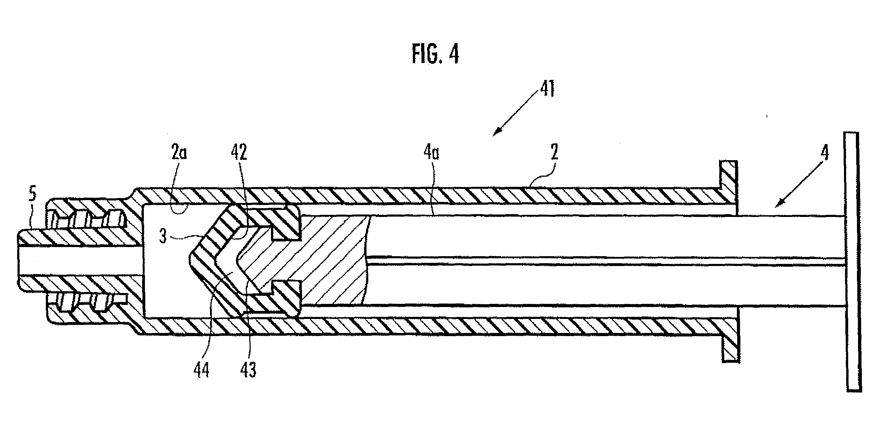

As a syringe used in medical practice including injection of drug solution into a body and sampling blood, the one shown in Figure 4 has been known. The syringe 41 shown in Figure 4 includes a barrel 2 with a lure tapered section 5 formed on the head, a gasket 3 that freely slides inside the barrel 2 to seal it, and a plunger rod 4 coupled to the gasket 3. The gasket 3 has a cavity section 42 at the inner periphery side. The plunger rod 4 is adapted to be coupled with the gasket 3 by fitting an anchor section 43 provided at the head to the cavity section 42.

The syringe 41 has the barrel 2 and the plunger rod 4, both of which are made of synthetic resin processed in injection molding. When they are made in that way, a certain clearance needs to be provided between the inner peripheral surface 2a of the barrel 2 and the outer peripheral surface 4a of the plunger rod 4. Without the clearance, it is hard for the plunger rod 4 coupled with the gasket 3 to be smoothly inserted into the barrel 2. The gasket 3 closely contacts the inner periphery surface 2a of the barrel 2 when it is attached to the head of the plunger rod 4 having the clearance between the inner periphery surface 2a of the barrel 2 and the outer peripheral surface 4a of the plunger rod 4, and prevents leakage of liquid or air when the gasket 3 slides along the inner periphery surface of the barrel 2 as the plunger rod 4 is operated.

As the syringe 41 has the clearance between the inner peripheral surface 2a of the barrel 2 and the outer peripheral surface 4a of the plunger rod 4, the plunger rod 4 cannot but sway in the axial direction of the barrel 2 when the plunger rod 4 is operated. If the gasket 3 sways with the plunger rod 4, the gasket 3 deforms and cannot keep close contact with the inner periphery side of the barrel 2, causing leakage of air or liquid.

Therefore, the syringe 41 is adapted to have a space 44 between the backside of the head of the gasket 3 and the head of the anchor section 43 when the anchor section 43 of the plunger 4 is fitted to the cavity section 42 provided at the inner periphery side of the gasket 3. That enables the anchor section 43 to move by taking advantage of the space 44 even if the plunger rod 4 sways as mentioned above. Therefore, the gasket 3 can keep the fitted state with the inner peripheral surface 2a of the barrel 2. The effect of the space 44 is quite effective in such a case in which a pressure to the gasket 3 is less than about 1.17 MPa such as when low-viscosity drug solution is injected into a body, or when blood is sampled.

Recently, a diagnosing method for observing blood vessels of the subject with X-ray apparatus or the like by injecting a contrast agent such as water soluble organic iodine chemical compound into the blood vessels is generally used. Under the diagnosing method, the contrast agent may be manually injected into the blood vessels using a syringe.

However, the contrast agent needs to be injected by the amount of 10 to 15 ml/second at the maximum to the weight of the subject, and is generally a high viscous liquid. Therefore, a pressure of 2.45 MPa or more is applied to the gasket 3 when the contrast agent is injected. In the syringe 41 shown in Figure 4, the gasket 3 depresses into the space 44 and deforms under the pressure, causing a problem in that a liquid leaks.

### Disclosure of the Invention

### Problems to be Solved by the Invention

The present invention intends to provide a syringe that eliminates the abovementioned problem and prevents occurrence of liquid leakage even if a high pressure is applied to a gasket.

### Means for Solving the Problem

In order to achieve the object, the syringe of the present invention comprising a barrel with a lure tapered section on the head, a gasket with a cavity section at an inner periphery side for freely sliding inside the barrel to seal the barrel, and a plunger rod with an anchor section projecting from the head to the gasket side to fit the cavity section, being arranged with a predetermined interval from the inner peripheral surface of the barrel, wherein the plunger rod is coupled with the gasket through the anchor section, characterized in that the anchor section has a head contact section that is projected from a pedestal section, which is formed at the head of the plunger rod, and contacts the backside of the gasket head, and a contact member that is loosely fitted on the outer periphery side of the anchor section and is in contact with the inner peripheral wall of the gasket, wherein the pedestal section has a tapered surface inclined at an angle in a range between 5 degrees and 45 degrees relative to a plane orthogonal to the axial direction of the plunger rod.

According to the syringe of the present invention, when the anchor section fits the cavity section of the gasket, the head contact section of the anchor section contacts the backside of the gasket head. Therefore, no space is formed between the anchor section and the backside of the gasket head. The contact member is loosely fitted on the outer periphery side of the anchor section so that the contact member contacts the inner peripheral wall. Accordingly, no space is formed between the contact member and the inner peripheral wall of the gasket.

Therefore, when a high pressure is applied to the gasket, the gasket is supported from inside by the head contact section of the anchor section and the contact member. That enables to prevent the gasket from deforming, preventing occurrence of liquid leakage.

However, a doctor injects a contrast agent in blood vessels of a subject for observing the blood vessels by an X-ray apparatus or the like. While watching a monitor or the like of the X-ray apparatus, the doctor may give insufficient attention to his/her hands. That easily causes the plunger rod to sway against the axial direction of the barrel when the doctor injects the contrast agent using the syringe. In such a case, in the syringe of the present invention, the head contact section of the anchor section contacts the backside of the gasket head as mentioned above, and the contact member contacts the inner peripheral wall of the gasket so that there is no space between the inner surface of the gasket and the anchor section or the contact member. As a result, when the plunger rod sways against the axial direction of the barrel, the gasket deforms following to the swaying. That may cause occurrence of liquid leakage.

The syringe of the present invention includes the contact member loosely fitted on the outer periphery side of the anchor section, which is projected from the pedestal section formed on the head of the plunger rod, with the pedestal section having a tapered surface inclined at an angle in a range between 5 degrees and 45 degrees relative to a plane orthogonal to the axial direction of the plunger rod. The pedestal section can make an interval between the surface of the pedestal section and the bottom of the contact member by having a tapered surface.

As a result, according to the syringe of the present invention, the plunger rod can sway in the axial direction of the barrel, while keeping the gasket closely contacting the inner peripheral surface of the barrel by an interval between the contact member and the anchor member, which are loosely fitted as mentioned above, and an interval formed by the tapered surface between the surface of the pedestal section and the bottom of the contact member. Accordingly, the syringe can prevent the gasket from deforming when the plunger rod sways so as to prevent occurrence of liquid leakage.

If the tapered surface is inclined at less than 5 degrees relative to a plane orthogonal to the axial direction of the plunger rod, it cannot form a sufficient interval between the surface of the pedestal section and the bottom of the contact member. As a result, it cannot obtain an effect of enabling the plunger rod to sway in the axial direction of the barrel, while keeping the gasket closely contacting the inner peripheral surface of the barrel. Also, even if the tapered surface is inclined to exceed 45 degrees relative to a plane orthogonal to the axial direction of the plunger rod, the abovementioned effect cannot be improved further.

Additionally, in the syringe of the present invention, the pedestal section is characterized by including a curved recess surface formed between the head contact section and the tapered surface, and the contact member has a curved projection section at a position facing the curved recess surface.

As the pedestal section has the curved recess surface and the contact member has the curved projection surface, the plunger rod can more easily sway in the axial direction of the barrel.

Additionally, the syringe of the present invention is characterized in that the barrel includes a lock tube having a screw thread formed on its inner periphery side that extends from the base of the lure tapered section along the axial direction of the lure tapered section. The syringe of the present invention can be connected with other members by screwing an end of the other member into a thread formed at the inner periphery side of the lock tube. The other member may include an injection needle with a base of a needle as the abovementioned end, and a connecting member with a plurality of three way stopcocks, for example.

The syringe of the present invention can prevent the gasket from modifying by supporting the gasket from inside by the anchor section and the contact member as mentioned above, and also allow the plunger to sway in the axial direction of the barrel, while keeping the gasket closely contacting the inner peripheral surface of the barrel. Therefore, the syringe can be preferably applied to a use in which a high pressure is applied to the gasket, for example, a use in which a contract agent with high-viscosity is injected into blood vessel of a subject.

### Brief Description of the Drawings

Figure 1 is an illustrative cross-sectional view showing a configuration of the syringe of the embodiment;
Figure 2 is an assembly drawing of the syringe shown in Figure 1;
Figure 3 is an enlarged view of main parts of the syringe shown in Figure 1; and
Figure 4 is an illustrative cross-sectional view showing an example of a configuration of a conventional syringe.

### Best Mode for Carrying Out the Invention

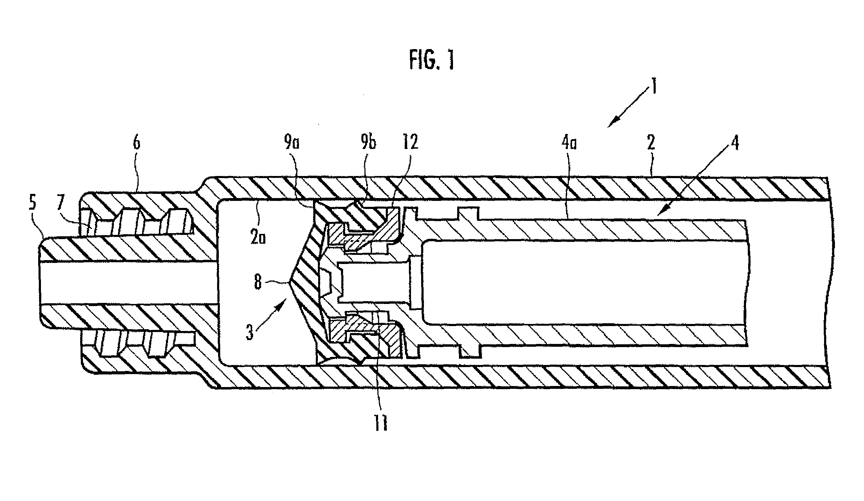

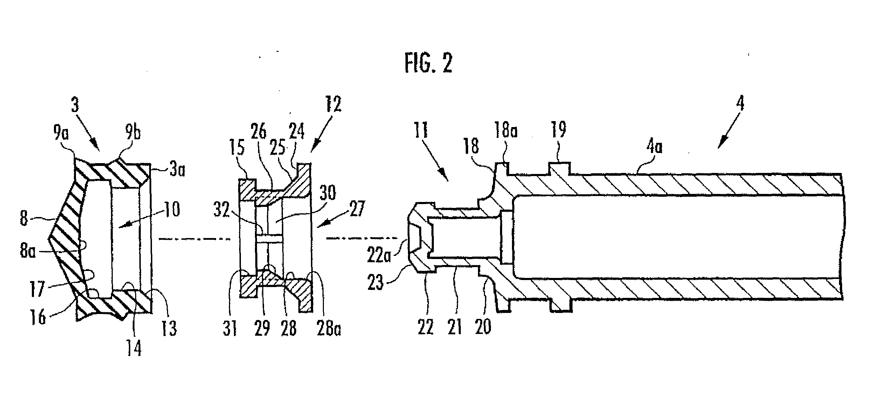

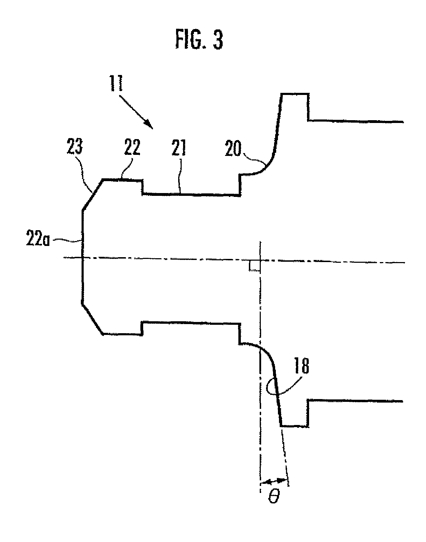

Now, an embodiment of the present invention will be described in further detail with reference to the drawings. Figure 1 is an illustrative cross-sectional view showing a configuration of the syringe of the embodiment. Figure 2 is an assembly drawing of the syringe shown in Figure 1. Figure 3 is an enlarged view of a substantial part of the syringe shown in Figure 1.

As shown in Figure 1, a syringe 1 of the embodiment includes a barrel 2, a gasket 3 arranged inside the barrel 2, and a plunger rod 4 coupled with the gasket 3. The barrel 2 and the plunger rod 4 are made of synthetic resin including polycarbonate resin, ABS resin (acrylonitrile-butadiene-styrene copolymer resin), and the like processed by injection molding. A clearance for enabling the plunger rod 4 to be smoothly inserted is provided between an inner peripheral surface 2a of the barrel 2 and an outer peripheral surface 4a of the plunger rod 4. The gasket 3 is preferably made of synthetic rubber such as silicon gum, for example, with the rubber hardness in a range between 50 to 70A (JIS K 6253).

The barrel 2 has a lure tapered section 5 formed at the head, and a lock tube 6 that extends from the base of the lure tapered section 5 along the axial direction of the lure tapered section 5. The lock tube 6 has a screw thread 7 inside. The syringe 1 can be connected with the injection needle, the connecting member or the like by screwing a base of a needle of the injection needle, an end of the connecting member with a plurality of three way stopcocks into a thread 7 of the lock tube 6, for example.

Next, the gasket 3 has a conical projection 8 formed at the head and two lines of circumferential projections 9a and 9b formed at the outer peripheral surface as shown in Figures 1 and 2, and a cavity section 10 at the inner periphery side. The circumferential projections 9a and 9b are in contact with the inner surface 2a of the barrel 2 so as to make the gasket 3 freely slide inside the barrel 2 to seal it.

The anchor section 11 projected from the head of the plunger rod 4 to the side of the gasket 3 and the ring-shaped contact member 12 that is loosely fitted to the outer periphery side of the anchor section 11 are fitted to the cavity section 10. The cavity section 10 includes a tapered section 13, which is tapered toward the head, for guiding the anchor section 11 and the contact member 12; a cylindrical section 14, which is continuously formed on the head of the tapered section 13; and an engaging section 16, which is continuously formed on the head of the cylindrical section 14 with the radius larger than that of the cylindrical section 14 for a circumferential projection section 15 that is formed at the head of the contact member 12 to be engaged therewith. In the gasket 3, the backside 8a of the conical projection 8 is flat and connected to the engaging section 16 through a tapered section 17.

Next, the plunger rod 4 includes a pedestal section 18 that is formed on the head and a circumferential projection section 19 formed at the rear side of the pedestal section 18, with the anchor section 11 projecting from the pedestal section 18 toward the gasket 3 as shown in Figures 1 and 2. The pedestal section 18 has a tapered surface inclined by the angle of θ relative to a plane orthogonal to the axial direction of the plunger rod 4, being tapered toward the head as shown in enlarged view in Figure 3. The angle is set within a range from 5 to 45 degrees, preferably within a range from 7 to 15 degrees, for example 8 degrees. The outer peripheral edge 18a of the pedestal section 18 is projected from the outer peripheral surface of the plunger rod 4.

The anchor section 11 includes a curved recess surface section 20 connected to the pedestal section 18, a cylindrical axial section 21 connected to the curved recess surface section 20, and the head contact section 22 formed on the head of the axial section 21, with the radius larger than that of the axial section 21 as shown in Figures 1 and 2. The curved recess surface section 20 has a radius around 0.8 to 2 mm, depending on the radius of the plunger 4. The head surface 22a of the head contact section 22 is flat and connected to the outer peripheral surface of the head contact section 22 through the tapered section 23.

Next, the contact member 12 includes a flange section 24 that contacts the back end section 3a of the gasket 3, a tapered contact surface 25 that is connected to the flange section 24 and contacts the tapered section 13 of the gasket 3, a cylindrical contact surface 26 that is connected with the tapered contact surface 25 and contacts the cylindrical section 14 of the gasket 3, and the circumferential projection section 15 that is connected with the cylindrical contact surface 26 with a radius larger than that of the cylindrical contact surface 26, and a through-hole 27 on the inner periphery side, as shown in Figures 1 and 2.

The through-hole 27 is adapted for the anchor section 11 to be inserted, and includes a larger radius section 28 formed at the side of the plunger rod 4, a tapered section 29 that is connected with the larger radius section 28 and tapered toward the head, a smaller radius section 30 that is connected with the tapered section 29, and a head opening section 31 with a radius intermediate between the larger radius section 28 and the smaller radius section 30 and that is connected to the smaller radius section 30. The larger radius section 28 is for accepting the tapered section 20 when the anchor section 11 is inserted. The larger radius section 28 has a curved projection surface 28a facing the curved recess surface 20 of the plunger 4 at the opening section at the side of the plunger rod 4. The curved projection surface 28a has a radius around 0.3 to 1 mm, depending on the radius of the plunger 4.

Additionally, the smaller radius section 30 is for loosely fitting the axial section 21 when the anchor section 11 is inserted. As the smaller radius section 30 has a radius smaller than that of the head contact section 22 of the anchor section 11, it has grooves 32 formed along the axial direction at four places equiangularly along the circumference for facilitating the head contact section 22 to be inserted. The head opening section 31 is adapted to accept the head contact section 22, and engage the head contact section 22 to a step section at the boundary with the smaller radius section 30 to fasten it when the anchor section 11 is inserted.

Now, operations of the syringe 1 of the embodiment will be described.

In the syringe 1 of the embodiment, the anchor section 11 of the plunger rod 4 is inserted into the through-hole 27 of the ring-shaped contact member 12. Here, the head contact section 22 formed on the head of the anchor section 11 is accepted into the head opening section 31 and also fastened by being engaged in the step at the boundary between the smaller radius section 30 and the head opening section, and there is a clearance between the outer peripheral surface of the head contact section 22 and the head opening section 31, and between the axial section 21 and the smaller radius section 30, respectively, as shown in Figures 1 and 2. Accordingly, the contact member 12 is loosely fitted on the outer periphery side of the anchor section 11 of the plunger rod 4. The head surface 22a and the tapered section 23 that form the leading edge of the anchor section 11 are projected from the head opening section 31 of the contact member 12 toward the gasket 3.

Next, the anchor section 11, with which the contact member 12 is loosely fitted as mentioned above, is fitted to the cavity section 10 of the gasket 3. At this time, at the head of the anchor 11, the head surface 22a of the head contact section 22 contacts the backside 8a of the conical projection 8 of the gasket 3 and the circumferential projection section 15 of the contact member 12 is engaged with the engaging section 16 of the cavity section 10 so that the gasket 3 is fastened to the anchor section 11. At the outer periphery side of the anchor section 11 (more specifically at the outer periphery side of the axis section 11), the cylindrical contact surface 26 of the contact member 12 contacts the cylindrical section 14 of the gasket 3, and the tapered contact surface 25 of the contact member 12 contacts the tapered section 13 of the gasket 3.

Accordingly, as almost all of the cavity section 10 closely attached with the anchor section 11 or the contact member 12, which is interposed between the cavity section 10 and the anchor section 11, the gasket 3 is supported from inside by the anchor section 11 and the contact member 12.

Additionally, the pedestal section 18 of the plunger rod 4 has a tapered surface inclined by the angle of θ relative to a plane orthogonal to the axial direction of the plunger rod 4, being tapered toward the head as mentioned above. Accordingly, a space with a cross-sectionally cuneiform shape is formed between the bottom of the contact member 12, which loosely fits the anchor section 11, and the surface of the pedestal section 18.

The syringe 1 of the embodiment is used for the purpose of, for example, injecting a contrast agent such as water-soluble organic iodine chemical compound into the blood vessel of a subject. At this moment, as the plunger rod 4 mounted with the gasket 3 is first inserted into the barrel 2, an end of the connecting member (not shown) with a plurality of three way stopcocks is connected with the screw thread 7 on the lock tube 6. Next, one of the three way stopcocks is opened and the plunger rod 4 is drawn so that the contrast agent such as water-soluble organic iodine chemical compound or the like is sucked in the barrel 2. After the three way stopcocks are closed, the plunger rod 4 is pressed so that the contrast agent is injected from a tube, an injection needle or the like that is connected with the other end of the connecting member into the blood vessel of the subject.

When the contrast agent is injected into the blood vessel of the subject, the pressure applied to the gasket 3 is increased to 2.45 MPa or more. As almost all of the cavity section 10 closely fits the anchor section 11 or the contact member 12, which is interposed between the cavity section 10 and the anchor section 11, the gasket 3 of the syringe 1 is supported from inside by the anchor section 11 and the contact member 12, so that the gasket 3 is not deformed by the pressure. This enables to prevent occurrence of the liquid leakage. A slight space like a part of the tapered section 17 of the cavity section 10 facing the tapered section 23 of the anchor section 11 may exist between the gasket 3 and the anchor section 11 or the contact member 12, but only in a range where the gasket 3 does not deform even under the high pressure.

Additionally, when a doctor injects the contrast agent into the blood vessel of the subject, the doctor watches a monitor or the like of the X-ray apparatus. Therefore, the doctor may give insufficient attention to his/her hands. As the clearance is provided between the inner peripheral surface 2a of the barrel 2 and the outer peripheral surface 4a of the plunger rod 4, if the doctor gives insufficient attention to the hands, the plunger rod 4 easily sways towards the axis direction of the barrel 2.

However, the clearance between the contact member 12 and the anchor member 11 that is provided as a result from the contact member 12 being loosely fitted with the outer periphery side of the anchor section 11, and a space between the bottom of the contact member 12 and the pedestal section 18 that is provided as the pedestal section 18 being the tapered surface, make the plunger rod 4 sway as mentioned above, while making the gasket 3 maintain close contact with the inner peripheral surface 2a of the barrel 2. As the plunger rod 4 has the curved recess surface 20 connected with the pedestal section 18 and the contact member 12 has the curved projection surface 28a facing the curved recess surface 20, the abovementioned swaying more easily occurs. Therefore, the syringe 1 can prevent deforming the gasket 3 even when the plunger rod 4 sways in the axial direction of the barrel 2, so that the occurrence of liquid leakage is prevented.

Further, as the plunger rod 4 has the circumferential projection section 19 on the outer peripheral surface 4a so that the circumferential projection section 19 contacts the inner peripheral surface 2a of the barrel 2 when the plunger rod 4 sways as mentioned above, the swaying can be restricted to small range.

## Claims

1. A syringe comprising a barrel with a lure tapered section on the head, a gasket with a cavity section at an inner periphery side for freely sliding inside the barrel to seal the barrel, and a plunger rod with an anchor section projecting from the head to the gasket side to fit the cavity section, being arranged with a predetermined interval from the inner periphery side of the barrel, wherein the plunger rod is coupled with the gasket through the anchor section, **characterized in that**
the anchor section has a head contact section that is projected from a pedestal section, which is formed at the head of the plunger rod, and contacts the backside of the gasket head, and a contact member that is loosely fitted on the outer periphery side of the anchor section and is in contact with the inner peripheral wall of the gasket, and
the pedestal section has a tapered surface inclined at an angle in a range between 5 degrees and 45 degrees relative to a plane orthogonal to the axial direction of the plunger rod.

2. The syringe according to claim 1, **characterized in that** said pedestal section has a curved recess surface formed between said head contact section and said tapered surface, and said contact member has a curved projection section at a position facing the curved recess surface.

3. The syringe according to claim 1, **characterized in that** said barrel has a lock tube that extends from the base of the lure tapered section along the axial direction of the lure tapered section with a screw thread on the inner periphery side.

4. The syringe according to claim 1, **characterized by** being used in injecting a contrast agent.
